# EUROPEAN PATENT APPLICATION

(11) **EP 1 449 823 A1**
(43) Date of publication of application: **25.08.2004**
(21) Application number: 02801528.7
(22) Date of filing: 10.10.2002
(51) Int. Cl.: C07C 69/34, C10M 105/36

(54) **DIBASIC ACID DIESTER**

(30) Priority: 10.10.2001 JP 2001312099
(71) Applicant: Kyowa Yuka Co., Ltd., Tokyo 100-8185 (JP)
(72) Inventor: FUKUDA, Yukitoshi, c/o Yokkaichi Plant, Yokkaichi-shi, Mie 510-8502 (JP); SHIMIZU, Ikuo, c/o Yokkaichi Research Laboratories, Yokkaichi-shi, Mie 510-8502 (JP); ITO, Katsuhiro, Yokkaichi Research Laboratories, Yokkaichi-shi, Mie 510-8502 (JP); OSADA, Kazuyasu, Yokkaichi Research Laboratories, Yokkaichi-shi, Mie 510-8502 (JP)
(74) Representative: Casalonga, Axel
(86) International application number: PCT/JP2002/010528
(87) International publication number: WO 2003/033451

(57) **Abstract**

The present invention provides dibasic acid diesters, which are suitable for use in lubricating base oils, etc. and excellent in hydrolysis resistance, etc., represented by general formula (I): 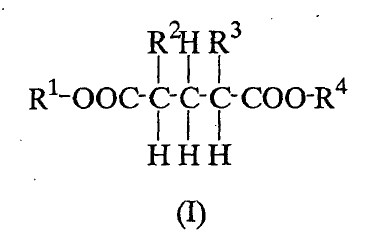 (wherein R² and R³, which may be the same or different, each represent lower alkyl; and R¹ and R⁴, which may be the same or different, each represent alkyl having 7 or more carbon atoms).

## Description

### Technical Field

The present invention relates to dibasic acid diesters suitable for use in lubricating base oils, etc.

### Background Art

As base oils for lubricating oils and grease, those having a high stability and a long life are desirable.

Known examples of the base oils for lubricating oils or grease include those comprising dibasic acid diesters. As the lubricating oil whose base oil is a dibasic acid diester, WO97/21792 discloses a lubricating oil whose base oil is an alicyclic polycarboxylic acid ester, and Japanese Published Unexamined Patent Application No. 2001-89776 discloses a lubricating oil for a freezer whose base oil is 1,2-cyclohexanedicarboxylic acid diester. However, neither of the above lubricating oils whose base oil is a polycarboxylic acid diester is practically satisfactory in respect of hydrolysis resistance.

### Disclosure of the Invention

An object of the present invention is to provide dibasic acid diesters, which are suitable for use in lubricating base oils, etc. and excellent in hydrolysis resistance, etc.

The present invention provides the following (1) to (4).
(1) A dibasic acid diester represented by general formula (I): 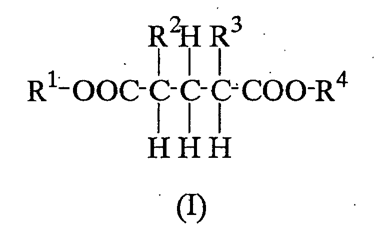 (wherein R² and R³, which may be the same or different, each represent lower alkyl; and R¹ and R⁴, which may be the same or different, each represent alkyl having 7 or more carbon atoms).
(2) The dibasic acid diester according to (1), wherein R¹ and R⁴ each are alkyl having 7 to 40 carbon atoms.
(3) A lubricating base oil comprising the dibasic acid diester according to (1) or (2).
(4) A lubricating oil comprising the lubricating base oil according to (3).

Hereinafter, the dibasic acid diesters represented by general formula (I) may be referred to as Compounds (I).

In the definitions of the groups in general formula (I), the lower alkyl includes straight-chain or branched alkyl groups having 1 to 8 carbon atoms, such as methyl, ethyl, propyl, isopropyl, butyl, isobutyl, sec-butyl, tert-butyl, pentyl, isoamyl, neopentyl, hexyl, heptyl, octyl, isooctyl and 2-ethylhexyl. As R² and R³, alkyl having 1 to 4 carbon atoms is preferred, and ethyl is more preferred.

The alkyl having 7 or more carbon atoms includes straight-chain or branched ones such as heptyl, octyl, 2-octyl, 3-octyl, isooctyl, 2-ethylhexyl, 4-methyl-3-heptyl, 2-propyl-1-pentyl, 2,4,4-trimethyl-1-pentyl, 2,2-dimethyl-3-hexyl, 2,3-dimethyl-2-hexyl, 2,5-dimethyl-2-hexyl, 2,5-dimethyl-3-hexyl, 3,4-dimethyl-3-hexyl, 3,5-dimethyl-3-hexyl, 3-ethyl-2-methyl-3-pentyl, 2-methyl-2-heptyl, 3-methyl-3-heptyl, 4-methyl-4-heptyl, 5-methyl-1-heptyl, 5-methyl-2-heptyl, 5-methyl-3-heptyl, 6-methyl-2-heptyl, 6-methyl-3-heptyl, nonyl, 2-nonyl, 3-nonyl, 4-nonyl, 5-nonyl, 2-methyl-3-octyl, 6-methyl-1-octyl, 3,5,5-trimethyl-1-hexyl, 2,6-dimethyl-4-heptyl, 3-ethyl-2,2-dimethyl-3-pentyl, decyl, 2-decyl, 3-decyl, 4-decyl, 5-decyl, 3,7-dimethyl-1-octyl, 3,7-dimethyl-3-octyl, undecyl, 2-undecyl, 3-undecyl, 4-undecyl, 5-undecyl, 6-undecyl, dodecyl, 2-dodecyl, 2-butyl-1-octyl, 2-tridecyl, tridecyl, tetradecyl, 2-tetradecyl, 7-tetradecyl, 7-ethyl-2-methyl-4-undecyl, pentadecyl, hexadecyl, 2-hexadecyl, 2-hexyl-1-decyl, heptadecyl, octadecyl (stearyl),'isostearyl, 5,7,7-trimethyl-2-(1,3,3-trimethylbutyl)octyl, nonadecyl, eicosyl, docosyl, 2-octyl-1-dodecyl, triacontyl and tetracontyl. R¹ and R⁴ are preferably alkyl having 7 to 40 carbon atoms, more preferably alkyl having 7 to 30 carbon atoms, further preferably alkyl having 7 to 25 carbon atoms, and particularly preferably alkyl having 7 to 20 carbon atoms.

Compounds (I) can be produced by known methods for synthesis of esters. For example, Compound (I) can be obtained by reacting the corresponding dibasic acid with 1 to 10 equivalents, preferably 1 to 2 equivalents of an aliphatic alcohol, if necessary, in the presence of an entrainer and a catalytic amount to 0.5 equivalent of an acid catalyst such as p-toluenesulfonic acid at 50 to 180°C. Examples of the entrainers include toluene and benzene. They are usually used in an amount of 0.5 to 100 equivalents on the basis of dibasic acid.

A dibasic acid used as a starting material can be produced by treating the corresponding diol in the presence of 1 to 5 equivalents of a base such as sodium hydroxide or potassium hydroxide, preferably at 200 to 320°C according to known methods [Yukagaku (oil Chemistry), Vol. 19, No. 12, p. 1087 (1970); Japanese Published Unexamined Patent Application No. 72948/94; etc.]. In this treatment, a reaction solvent such as an ether solvent (e.g., dibenzyl ether) or a hydrocarbon solvent (e.g., liquid paraffin having 10 to 16 carbon atoms) may be used.

The lubricating base oil of the present invention preferably contains Compound (I) in an amount of 10 wt% or more, more preferably 25 wt% or more, further preferably 50 wt% or more on the basis of the total amount.

The lubricating base oil of the present invention may contain additional base oils such as ester oils, poly-α-olefin, mineral oils and silicone oils as may be required.

Examples of the ester oils are fatty acid monoesters, adipic acid diesters, azelaic acid diesters, sebacic acid diesters and phthalic acid diesters.

Examples of the poly-α-olefin are low molecular weight polybutene, low molecular weight polypropylene'and α-olefin oligomers having 8 to 14 carbon atoms.

Examples of the mineral oils are paraffin-base crude oil, intermediate base crude oil and naphthene-base crude oil.

There is no specific restriction as to the amount of additional base oils such as ester oils, poly-α-olefin, mineral oils and silicone oils, but it is preferably 90 wt% or less, more preferably 50 wt% or less on the basis of Compound (I).

The lubricating oil of the present invention can be obtained by adding to the lubricating base oil of the present invention, according to need, additives such as a detergent-dispersant, an antioxidant, an extreme-pressure additive, a rust inhibitor, a vapor phase rust inhibitor, a pour point depressant, a thickener, an antiseptic, an antifoaming agent, a demulsifier, an extreme-pressure additive, a dye and a perfume. There is no specific restriction as to the amount of these additives, but it is preferably 0.01 to 5 wt% of the lubricating oil of the present invention.

The lubricating base oil and the lubricating oil of the present invention are excellent in hydrolysis resistance, lubrication, heat resistance, low temperature fluidity, flame resistance, biodegradability, or the like.

The lubricating oil of the present invention can be employed, for example, as engine oil, turbine oil, hydraulic oil, refrigerating oil, rolling oil, grease, lubricating oil for metal processing, etc. according to known methods (WO97/21792, Japanese Published Unexamined Patent Application No. 2001-89776, etc.).

### Test Example 1 Hydrolysis Resistance Test

Hydrolysis resistance test was carried out using Compounds 1 and 2 respectively synthesized in Examples 1 and 2, and DOA (dioctyl adipate) as a comparative compound. Test method: Into a test solution comprising a test sample and water (weight ratio: 3:1) were put a copper piece and an iron piece as catalysts, and the test solution was allowed to stand with stirring at 100°C for 168 hours. The total acid number of the oil layer was measured after standing (the total acid number becomes larger as hydrolysis proceeds). The test results are shown in Table 1.

**Table 1**

| | Total acid value (mgKOH/g) |
|---|---|
| Compound 1 | 0.12 |
| Compound 2 | 0.03 |
| Comparative compound | 0.26 |

It is evident from Table 1 that Compounds 1 and 2 are remarkably excellent in hydrolysis resistance, compared with the comparative compound.

### Best Modes for Carrying Out the Invention

Measurement data in the following reference example and examples were obtained using the following measurement instruments.
Mass spectrum (MS): M-80B mass spectrometer (Hitachi Co., Ltd.)
Infrared spectrum (IR): FTS-40A (Bio-Rad Japan)
Proton nuclear magnetic resonance spectrum (¹H-NMR): GSX-400 (400 MHz) (JEOL Ltd.)
Density: electronic densimeter SP-120L (Alfa Mirage Co., Ltd.)
Kinematic viscosity: Cannon-Fenske viscometer (measured according to the method of JIS K2283)

### Reference Example 1

2,4-Diethyl-1,5-pentanediol (160.3 g) (trade name: Kyowadiol PD-9, Kyowa Yuka Co., Ltd., purity: 93.9%), 156.6 g of potassium hydroxide (purity: 86%) and 102.2 g of carbon number 12 paraffin mixture (trade name: Kyowasol C1200-H, Kyowa Yuka Co., Ltd.) were placed in a 1L nickel autoclave equipped with a reflux condenser, a pressure control valve and an electric furnace capable of temperature control, and heated with stirring under 1 MPa. The generated hydrogen gas was measured with a gas meter, and the progress of reaction was monitored. The generation of the gas was confirmed at around 230°C, and the reaction was continued at a temperature maintained in the range of 250 to 270°C. After the temperature reached 250°C, 89.4 l of hydrogen was generated in 3.5 hours. The reaction was continued for further 30 minutes, during which 0.8 l of hydrogen gas was generated. The amount of the generated hydrogen agreed with the theoretical value and the rate of reaction was 100%. After the reaction, the reaction mixture containing dipotassium 2,4-diethylglutarate was dissolved in water, and sulfuric acid was added thereto. The deposited solid was separated therefrom by filtration to obtain crude 2,4-diethylglutaric acid. The obtained crude 2,4-diethylglutaric acid was washed with water and purified by crystallization from n-hexane, whereby 142.5 g of 2,4-diethylglutaric acid (white crystals) was obtained. The obtained 2,4-diethylglutaric acid had 98.3% purity (calculated from acid value) (yield: 79.1%).

### Example 1 Synthesis of bis(2-ethylhexyl) 2,4-diethylglutarate (Compound 1)

In a reaction flask were placed 2,4-diethylglutaric acid (92.50 g), 2-ethylhexanol (66.00 g) and toluene (157.3 g), and the mixture was stirred well and p-toluenesulfonic acid monohydrate (2.67 g) was added thereto, followed by reflux for 5 hours. After cooling to room temperature, the reaction mixture was neutralized with a 0.1 wt% aqueous solution of sodium hydroxide and then washed with water. The solvent was distilled away from the reaction mixture at 135°C in vacuo, whereby 146 g of Compound 1 (yield: 98.4%) was obtained. The physical properties of Compound 1 were as follows.
¹H-NMR (CDCl₃, δ ppm): 3.98 (m, 4H), 2.32 (m, 2H), 1.94 (m, 1H), 1.75 (m, 1H), 1.59 (m, 4H), 1.28 (m, 18H), 0.95 (m, 18H)
IR (cm⁻¹): 2972, 2942, 2887 (C-H), 1745 (C=O), 1465, 1388, 1261, 1163 (C-O)
MS (m/z): 414 (M⁺)
Density (kg/m³): 929 (25°C)
Kinematic viscosity (cSt): 9.47 (40°C), 2.53 (100°C)

### Example 2 Synthesis of bis[5,7,7-trimethyl-2-(1,3,3-trimethylbutyl)octyl] 2,4-diethylglutarate (Compound 2)

In a reaction flask were placed 2,4-diethylglutaric acid (92.06 g), 5,7,7-trimethyl-2-(1,3,3-trimethylbutyl)octyl alcohol (28.32 g) and toluene (30.23 g), and the mixture was stirred well and p-toluenesulfonic acid monohydrate (1.13 g) was added thereto, followed by reflux for 4 hours. After cooling to room temperature, the reaction mixture was neutralized with a 0.2 wt% aqueous solution of sodium hydroxide and then washed with water. 5,7,7-Trimethyl-2-(1,3,3-trimethylbutyl)octyl alcohol was removed from the reaction mixture by extraction with methanol (three times), whereby 82.9 g of Compound 2 (yield: 79.0%) was obtained. The physical properties of Compound 2 were as follows.
¹H-NMR (CDCl₃, δ ppm): 4.01 (m, 4H), 2.32 (m, 2H), 1.96-1.04 (m, 28H), 0.88 (m, 54H)
IR (cm⁻¹): 2962, 2916, 2887 (C-H), 1743 (C=O), 1471, 1384, 1233, 1197, 1164 (C-O)
Density (kg/m³): 879 (26°C)
Kinematic viscosity (cSt): 270 (40°C), 14.3 (100°C)

### Industrial Applicability

The present invention provides dibasic acid diesters, which are suitable for use in lubricating base oils, etc. and excellent in hydrolysis resistance, etc.

## Claims

1. A dibasic acid diester represented by general formula (I): 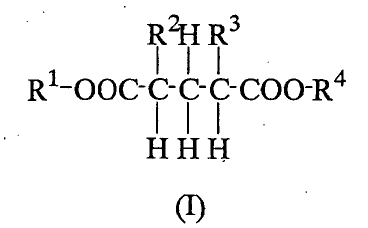 (wherein R² and R³, which may be the same or different, each represent lower alkyl; and R¹ and R⁴, which may be the same or different, each represent alkyl having 7 or more carbon atoms).

2. The dibasic acid diester according to Claim 1, wherein R¹ and R⁴ each are alkyl having 7 to 40 carbon atoms.

3. A lubricating base oil comprising the dibasic acid diester according to Claim 1 or 2.

4. A lubricating oil comprising the lubricating base oil according to Claim 3.
